# DEMANDE DE BREVET EUROPEEN

(11) **EP 0 787 467 A1**
(43) Date de publication de la demande: **06.08.1997**
(21) Numéro de dépôt: 97400258.6
(22) Date de dépôt: 05.02.1997
(51) Int. Cl.: A61B 17/86

(54) **Dispositif protecteur pour broche d'implantation osseuse**

(30) Priorité: 05.02.1996 FR 9601350
(71) Demandeur: Kehyayan, Georges, 92140 Clamart (FR); Choukroun, René, 75016 Paris (FR)
(72) Inventeur: Kehyayan, Georges, 92140 Clamart (FR); Choukroun, René, 75016 Paris (FR)
(74) Mandataire: Bruder, Michel

(57) **Abrégé**

La présente invention concerne un dispositif protecteur destiné à être enfilé sur une broche sécable (3) de façon à assurer l'immobilisation de celle-ci dans une pièce osseuse (1).

Ce dispositif est caractérisé en ce qu'il comporte un élément de semi-blocage (9) apte à coulisser dans un sens (A) sur la broche (3) à savoir dans la direction de la pièce osseuse (1) et à être bloqué sur celle-ci dans l'autre sens, et au moins une partie élastique (7b) permettant à la face postérieure du dispositif de se déplacer vers la pièce osseuse (1) sous l'action d'une sollicitation afin d'autoriser le sectionnement de la broche (3), et de reprendre sa position initiale lorsque ladite sollicitation cesse, afin de masquer la partie sectionnée (15) de la broche (3).

## Description

La présente invention concerne un dispositif protecteur des broches utilisées dans le domaine de l'implantation osseuse.

Dans certaines interventions chirurgicales le chirurgien utilise une broche métallique qu'il enfonce dans des parties osseuses d'un patient pour en assurer l'immobilisation temporaire. Une fois la broche introduite, le chirurgien cisaille la partie externe de celle-ci avec une pince de façon à l'adapter à la longueur souhaitée. Cette technique opératoire est susceptible d'entraîner deux inconvénients notoires.

Un premier inconvénient réside dans la possibilité de migration de la broche dans le sens de l'enfoncement, au travers des parties osseuses, sous l'effet des divers mouvements effectués par le patient, enfoncement qui risque, lorsque la broche ressort sur la paroi opposée des parties osseuses, de provoquer des lésions graves de certains éléments tissulaires. Un second inconvénient provient du caractère contondant de l'extrémité cisaillée de la broche qui risque de causer des dommages aux tissus environnants.

C'est pourquoi on a proposé, dans l'état antérieur de la technique, de coiffer la partie cisaillée de la broche d'un embout qui, d'une part, assure la stabilisation de celle-ci à l'encontre de toute migration dans les éléments osseux et, d'autre part, assure la protection des tissus environnants.

On a ainsi proposé, dans l'état antérieur de la technique, différents dispositifs de fixation d'un embout sur cette broche.

On a ainsi proposé de faire appel à une broche filetée sur laquelle l'embout vient se fixer par vissage après le cisaillage de celle-ci. La mise en oeuvre d'une telle technique présente un certain nombre de difficultés. Tout d'abord l'opération de cisaillage de la broche provoque la formation d'une bavure et une déformation du filetage, si bien qu'il est particulièrement difficile par la suite de venir visser l'embout sur la broche filetée. De plus, la mise en place de l'embout sur la broche nécessite un repérage préalable de l'extrémité de celle-ci, repérage qui est particulièrement difficile à effectuer au travers d'une incision cutanée la plupart du temps de dimensions réduites.

On a également proposé d'utiliser une broche lisse, et de coiffer celle-ci d'une olive qui est pourvue d'une vis transversale permettant de la fixer sur la broche. Ce dispositif présente l'inconvénient d'être d'une mise en oeuvre difficile en raison notamment des faibles qualités de préhension de l'olive, qui résultent notamment de ses dimensions réduites, et de la présence de la bavure formée sur la broche lors du cisaillage de celle-ci. De plus, son vissage latéral est difficile à mettre en oeuvre puisque l'extrémité cisaillée de la broche est plus ou moins enfouie dans les tissus environnants ce qui rend difficile l'accès à la vis de serrage.

Afin d'améliorer la préhension de l'embout que l'on souhaite disposer en extrémité de broche, on a proposé de solidariser celui-ci d'un organe de préhension permettant à la fois de le mettre en place et de commander la rotation d'une vis permettant de le fixer sur la broche. Si un tel dispositif a permis d'améliorer sensiblement la mise en place de l'embout il n'a cependant pu résoudre tous les problèmes liés à une telle mise en place. En effet l'organe de préhension n'est pas disposé dans l'axe de l'embout qui doit coïncider avec celui de la broche, si bien qu'il est difficile au chirurgien, dans des conditions opératoires parfois difficiles, d'aligner parfaitement ces deux axes avant la mise en place. De plus, aussi bien dans ce dispositif que dans les dispositifs précédents, la présence de la bavure perturbe la mise en place correcte de l'embout.

La présente invention a pour but de remédier à ces inconvénients en proposant un dispositif de blocage qui évite le problème de l'enfilage de l'embout sur la broche et celui créé par la présence de bavures au niveau de la ligne de coupe de celle-ci. La présente invention permet en effet d'assurer l'enfilage de l'embout sur la broche avant le cisaillage de celle-ci, ce qui supprime tout problème de mise en place ultérieur.

Par ailleurs la présente invention permet d'utiliser la broche pour guider de manière automatique l'embout protecteur jusqu'au contact de la partie osseuse sur laquelle il vient en butée, ce qui a pour effet de simplifier ainsi à l'extrême, la mise en place du dispositif.

La présente invention a ainsi pour objet un dispositif protecteur destiné à être enfilé sur une broche de façon à assurer l'immobilisation de celle-ci dans une pièce osseuse, caractérisé en ce qu'il comporte:
- un élément de semi-blocage apte à coulisser dans un sens sur la broche, à savoir dans la direction de la pièce osseuse, et à être bloqué sur celle-ci dans l'autre sens,
- au moins une partie élastique permettant à sa face postérieure de se déplacer vers la pièce osseuse sous l'action d'une sollicitation, afin d'autoriser le sectionnement de la broche, et de reprendre sa position initiale lorsque ladite sollicitation cesse, afin de masquer la partie sectionnée de la broche.

Dans un mode de mise en oeuvre de l'invention la partie élastique est disposée à l'opposé de l'extrémité destinée à venir en contact avec la pièce osseuse lors de la mise en place.

Dans un autre mode de mise en oeuvre de l'invention la partie élastique est pourvue d'une cavité interne.

Dans un autre mode de mise en oeuvre de l'invention l'élément de semi-blocage est noyé dans le même matériau que celui constituant la partie élastique. Cet élément de semi-blocage peut comporter au moins une lèvre inclinée de l'amont vers l'aval et de la broche vers l'extérieur.

On décrira ci-après, à titre d'exemples non limitatifs, diverses formes d'exécution de la présente invention, en référence au dessin annexé sur lequel :

La figure 1 est une vue en coupe axiale d'une pièce osseuse dans laquelle vient d'être implantée une broche équipée d'un dispositif protecteur suivant l'invention.

La figure 2 est une vue en coupe axiale de la pièce osseuse et du dispositif protecteur représentés sur la figure 1, au cours de la phase de cisaillage de la broche.

La figure 3 est une vue en coupe axiale de la pièce osseuse et du dispositif protecteur représentés sur les figures 1 et 2 une fois la mise en place terminée.

La figure 4 est une vue en coupe axiale d'une variante de mise en oeuvre de l'invention.

Les figures 5a et 5b sont deux vues en coupe axiale d'une variante de mise en oeuvre de l'invention respectivement avant et après cisaillage de la broche.

Les figures 6 et 7 sont des vues en coupe axiale de deux variantes de mise en oeuvre de l'invention.

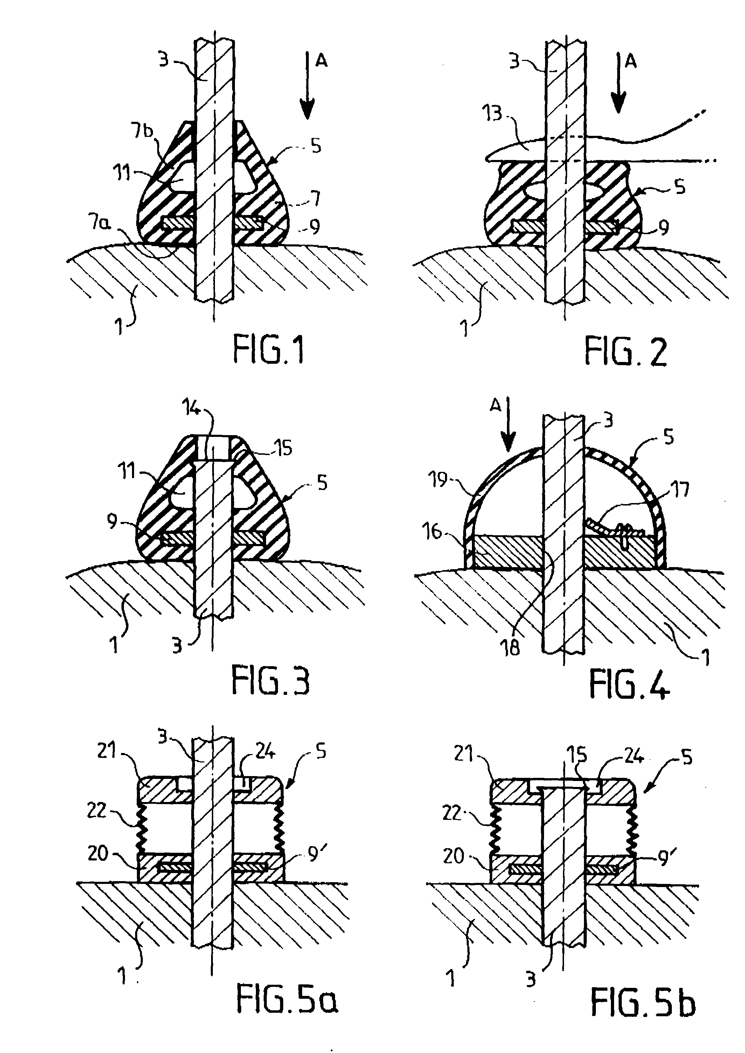

On a représenté sur la figure 1 une pièce osseuse 1 dans laquelle est implantée une broche métallique de maintien provisoire 3. Un embout 5, suivant l'invention, est enfilé sur la broche 3. Cet embout est constitué d'une enveloppe 7 réalisée dans un matériau élastique, tel que notamment un élastomère, compatible biologiquement avec les tissus environnants. Cette enveloppe 7 a la forme globale d'un tronc de cône dont les angles sont arrondis, la base 7a du tronc de cône étant appliquée contre la surface externe de la pièce osseuse 1. A l'intérieur de l'enveloppe 7, à proximité de sa base 7a, une rondelle frein 9 est noyée dans le matériau élastique. Cette rondelle frein 9 est d'un type tel que tout effort exercé sur l'embout 5 dans le sens de la flèche A, c'est-à-dire traduisant un effort ayant pour effet d'appliquer la base 7a de l'enveloppe 7 contre la surface externe de la pièce osseuse 1, a pour effet de faire avancer l'embout 5 sur la broche 3. Par contre, tout effort exercé en sens inverse sur l'embout 5 n'aura aucun effet de déplacement de celui-ci. Cette rondelle frein peut être du type dit rondelle "d'arrêt d'axe". L'extrémité 7b de l'embout 5, opposée à celle destinée à venir en contact avec la pièce osseuse 1, qui est en amont de la rondelle 9, (si l'on considère le sens de déplacement possible A de l'embout 5) est élastique et peut donc être déformée temporairement sous l'action d'un effort de direction A. La partie supérieure de l'embout 5 comporte une cavité interne 11.

Dans ces conditions la mise en oeuvre du dispositif protecteur suivant l'invention se fait ainsi que décrit ci-après. Avant son intervention, le chirurgien enfile l'embout 5 sur la broche 3 puis met celle-ci en place d'une façon identique à celle qu'il utilise habituellement. Le chirurgien fait alors coulisser l'embout 5 sur la broche 3 afin d'amener la face 7a de l'enveloppe 7 en contact avec la pièce osseuse 1. On se trouve alors dans la phase de mise en oeuvre illustrée par la figure 1.

Ensuite, comme représenté sur la figure 2, le chirurgien appuie avec une pince coupante 13 sur la partie supérieure élastique 7b de l'embout 5, dans le sens de la flèche A, afin de l'appliquer fortement sur la partie osseuse 1, et de comprimer sa partie supérieure afin de la déformer en la rapprochant de la partie osseuse 1. Le chirurgien cisaille ensuite la broche 3 avec la pince 13. Comme exposé précédemment, le mode de coupe a pour effet de créer, à l'extrémité cisaillée 14 de la broche 3, une bavure 15.(Figure 3)

Une fois la pince coupante 13 retirée, la matière élastique dont est constituée la partie supérieure 7b de l'embout 5 reprend sa forme initiale, comme représenté sur la figure 3, si bien que l'extrémité cisaillée 14 de la broche 3 ainsi que la bavure de cisaillage 15 se trouvent désormais à l'intérieur de l'embout 5.

Dans cette position, la broche 3 ne peut pénétrer plus avant dans la pièce osseuse 1 sous l'effet des mouvements du patient, puisque l'embout 5 est empêché de reculer sur la broche 3 par la rondelle 9. De plus, les tissus entourant l'extrémité coupée 14 de la broche 3 sont protégés du caractère contondant de celle-ci et de la bavure 15 par l'embout 5.

La présente invention est particulièrement intéressante en ce qu'elle permet notamment de réaliser à la fois le cisaillage de la broche 3 et la mise en place de la butée 5 "en aveugle" c'est à dire sans que le chirurgien soit obligé de voir l'extrémité cisaillée 14 de la broche 3 puisque c'est la broche 3 elle-même qui assure le guidage en position de l'embout 5. Le présent dispositif permet ainsi de réaliser de telles opérations de façon percutanée ce qui est beaucoup moins traumatisant pour le patient.

Bien entendu l'embout 5 peut être réalisé de multiples façons. Ainsi que représenté sur la figure 4, il peut être constitué d'un socle cylindrique rigide 16, en matière plastique ou en métal, percé en son centre d'un trou 18 destiné à recevoir la broche 3. Une lamelle métallique 17 est fixée sur la surface supérieure du socle 16, de façon qu'elle soit appliquée contre la broche 3 lorsque celle-ci est enfilée dans le trou 18 du socle 16. Cette lamelle 17 s'étend sur la figure 4 du haut vers le bas et du centre vers la périphérie, de façon que l'embout 5 ne puisse reculer par rapport à la broche 3 et s'éloigner de la pièce osseuse 1, mais puisse par contre facilement se rapprocher de celle-ci.

Une membrane élastique semi-sphérique 19 est fixée sur la périphérie du socle 16. Cette membrane 19 peut se déformer vers l'intérieur lorsque, comme sur la figure 2, elle est repoussée dans le sens de la flèche A vers la pièce osseuse 1, par la pince 13 du chirurgien, ce qui permet à celui-ci de cisailler la broche 3 à la distance requise et de voire celle-ci recouverte ensuite par la membrane 19.

Bien entendu, la partie élastique du dispositif protecteur suivant l'invention peut être située ailleurs qu'à l'extrémité opposée à celle destinée à venir en contact avec la pièce osseuse 1.

Ainsi, sur les figures 5a et 5b on a représenté, sous forme schématique, un embout 5 dont l'extrémité aval c'est-à-dire celle destinée à venir en contact avec la pièce osseuse 1 et l'extrémité amont c'est-à-dire l'extrémité opposée, sont constituées d'un disque rigide respectivement 20 et 21. Le disque 20 est pourvu de moyens de semi-blocage 9', au sens défini précédemment, et le disque 21 est creusé d'une cuvette supérieure 24. Les deux disques 20 et 21 sont réunis par un soufflet élastique 22 disposé en amont des moyens de semi-blocage 9'. La mise en oeuvre s'effectue, comme expliqué précédemment en regard des figures 1 à 4 et, une fois la broche 3 cisaillée, l'extrémité 14 cisaillée de la broche 3 et la bavure 15 de celle-ci se trouvent disposées à l'intérieur de la cuvette 24.

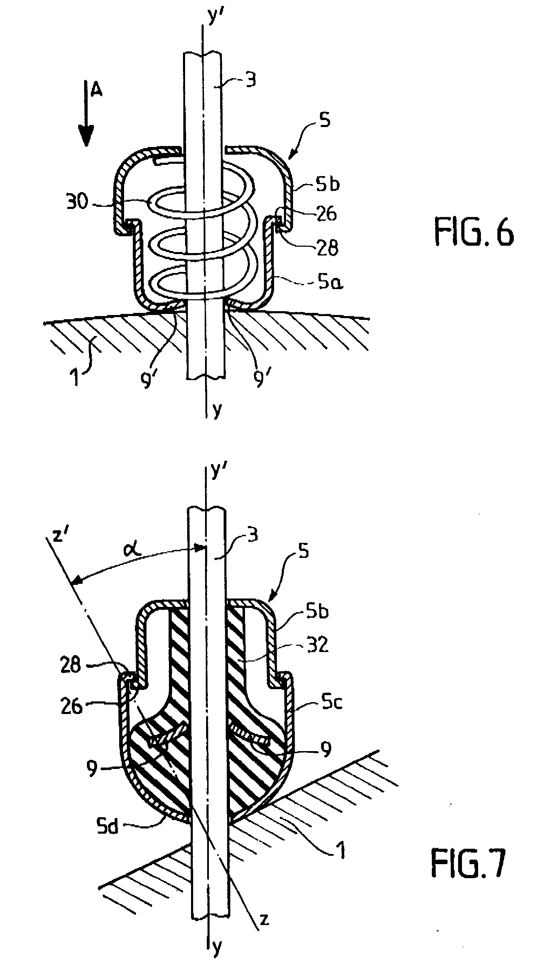

Le dispositif protecteur suivant l'invention peut également comporter une enveloppe métallique, ainsi que décrit dans les deux modes de mise en oeuvre représentés sur les figures 6 et 7.

Sur la figure 6, l'embout 5 est constitué d'un boîtier métallique cylindrique formés de deux coupelles 5a et 5b qui sont montées en vis à vis, à savoir une coupelle inférieure 5a dont le fond est destiné à venir en contact avec la pièce osseuse 1, et une coupelle supérieure 5b qui recouvre la coupelle 5a et qui est montée coulissante sur la face externe de celle-ci. La coupelle supérieure 5b comporte une butée circulaire interne 26 et la coupelle inférieure 5a comporte une butée circulaire externe 28, ces deux butées étant appliquées l'une contre l'autre sous la sollicitation créée par un ressort de compression 30 qui est disposé à l'intérieur des deux coupelles 5a, 5b. Ces dernières sont percées d'orifices destinés à permettre à une broche métallique 3 d'axe longitudinal yy' de les traverser de part en part.

Le fond de la coupelle 5a est relevé vers l'intérieur de façon à former des lèvres 9' destinées à venir en contact avec la broche 3 de façon à autoriser tout déplacement vers l'avant (sens de la flèche A) et interdire tout mouvement vers l'arrière (sens opposé à la flèche A) de l'embout 5 par rapport à la broche 3.

Le fonctionnement du présent dispositif est identique à celui des dispositifs décrits précédemment. Par rapport à ces derniers sa structure métallique améliore la résistance de l'embout aux agressions tant chimiques que mécaniques. Le ressort 30 pourrait bien entendu être remplacé par tout autre moyen élastique et notamment par un élément déformable élastique par exemple en silicone.

Ainsi dans le mode de mise en oeuvre représenté sur la figure 7 l'embout 5 est également constitué de deux coupelles métalliques, à savoir une coupelle supérieure 5b et une coupelle inférieure 5c, cette dernière étant pourvue d'un fond 5d de forme semi-sphérique. Les butées circulaires 26 et 28 dont sont pourvues les deux coupelles sont appliquées l'une contre l'autre par un élément élastique 32 constitué d'un élastomère, tel que notamment un élément de silicone, qui prend appui à la fois sur les fonds respectifs des deux coupelles 5b et 5c. Un volume vide est prévu entre l'élément élastique 32 et les parois des coupelles 5b et 5c de façon à permettre d'une part le déplacement vers la partie osseuse 1 de la coupelle supérieure 5b, et la déformation de l'élément élastique 32 au cours de ce déplacement. On a noyé dans la masse de l'élément élastique 32 une rondelle frein 9 destinée, comme précédemment, à autoriser l'avancement et empêcher le recul de l'embout 5 sur la broche 3.

Le présent mode de mise en oeuvre est particulièrement intéressant en ce qu'en plus des avantages de l'invention précédemment mentionnés il permet au chirurgien, en raison de la forme inférieure arrondie de l'embout, de donner à la broche 3 un angle d'incidence a par rapport à l'axe zz' perpendiculaire à la pièce osseuse 1.

Le dispositif protecteur suivant l'invention pourrait bien entendu posséder également une partie supérieure métallique, améliorant la protection de l'embout, et une partie inférieure élastique conférant à celui-ci la déformabilité qui est nécessaire à la mise en oeuvre de l'invention.

## Revendications

1. Dispositif protecteur destiné à être enfilé sur une broche sécable (3) de façon à assurer l'immobilisation de celle-ci dans une pièce osseuse (1), caractérisé en ce qu'il comporte :
- un élément de semi-blocage (9,9',16,17) apte à coulisser dans un sens (A) sur la broche (3) à savoir dans la direction de la pièce osseuse et à être bloqué sur celle-ci dans l'autre sens,
- au moins une partie élastique (7b,22,30,32) permettant à la face postérieure du dispositif de se déplacer vers la pièce osseuse (1) sous l'action d'une sollicitation afin d'autoriser le sectionnement de la broche (3), et de reprendre sa position initiale lorsque ladite sollicitation cesse, afin de masquer la partie sectionnée (15) de la broche (3).

2. Dispositif suivant la revendication 1 caractérisé en ce que la partie élastique (7b,22) est disposée à l'opposé de l'extrémité (7a) destinée à venir en contact avec la pièce osseuse (1) lors de la mise en place.

3. Dispositif suivant l'une des revendications précédentes caractérisé en ce que la partie élastique est pourvue d'une cavité interne (11).

4. Dispositif suivant l'une des revendications précédentes caractérisé en ce que l'élément de semi-blocage (9) est noyé dans un matériau constituant la partie élastique.

5. Dispositif suivant l'une des revendications précédentes caractérisé en ce que l'élément de semi-blocage (9,16) comporte une rondelle frein du type dit "arrêt d'axe".

6. Dispositif suivant l'une des revendications précédentes caractérisé en ce que l'élément de semi-blocage comporte au moins une lèvre (17) inclinée de l'amont vers l'aval et de la broche (3) vers l'extérieur.

7. Dispositif suivant la revendication 1 caractérisé en ce qu'il est constitué de deux coupelles rigides (5a, 5b, 5c) montées coulissantes l'une par rapport à l'autre et formant entre elles une cavité, traversée par la broche (3), à l'intérieur de laquelle sont disposés des moyens élastiques (30,32) sollicitant les deux coupelles (5a, 5b, 5c) pour les éloigner l'une de l'autre.

8. Dispositif suivant la revendication 7 caractérisé en ce que les moyens élastiques sont constitués d'un ressort de compression (30).

9. Dispositif suivant la revendication 7 caractérisé en ce que les moyens élastiques sont constitués d'un élément (32) en élastomère.
